# EUROPÄISCHE PATENTANMELDUNG

(11) **EP 2 893 908 A1**
(43) Veröffentlichungstag der Anmeldung: **15.07.2015**
(21) Anmeldenummer: 15151057.5
(22) Anmeldetag: 14.01.2015
(51) Int. Cl.: A61F 5/01

(54) **Handgelenkorthese**

(30) Priorität: 14.01.2014 DE 102014100359
(71) Anmelder: Akademie für Handrehabilitation GmbH & Co. KG, 31848 Bad Münder (DE)
(72) Erfinder: Zumhasch, Rainer, 32676 Lüdge (DE); Klausch, Sven, 31789 Hameln (DE); Paries, Cornelia, 13503 Berlin (DE)
(74) Vertreter: Kröncke, Rolf

(57) **Zusammenfassung**

Die vorliegende Anmeldung richtet sich am ersten Aspekt auf eine Handgelenkorthese mit formstabiler Unterarmschiene (1) und formstabiler palmar anlegbarer Handschiene (2), die Bewegungen im Handgelenk in einem gewissen Umfang erlauben. Die Handgelenkorthese ist dabei derart ausgestaltet, dass die Unterarmschiene und die Handschiene um eine Drehachse (4) drehgelenkig miteinander verbunden sind und die Schienen relativ zueinander um einen Drehwinkel von maximal 20 Grad um die Drehachse beweglich sind. Solche Handgelenkorthesen sind besonders geeignet zur konservativen und postoperativen Therapie von ligamentären Verletzungen einschließlich ligamentären Rupturen. Die erfindungsgemäße Handgelenkorthese zeichnet sich dadurch aus, dass sie eine Bewegungsfreiheit der Hand relativ zum Unterarm erlaubt, wobei diese Bewegungsfreiheit derart begrenzt ist, dass die Sehnen und Bänder im Wesentlichen nicht belastet werden.

## Beschreibung

Die vorliegende Anmeldung richtet sich am ersten Aspekt auf eine Handgelenkorthese mit formstabiler Unterarmschiene und formstabiler palmar anlegbarer Handschiene, die Bewegungen im Handgelenk in einem gewissen Umfang erlauben. Die Handgelenkorthese ist dabei derart ausgestaltet, dass die Unterarmschiene und die Handschiene um eine Drehachse drehgelenkig miteinander verbunden sind und die Schienen relativ zueinander um einen Drehwinkel von maximal 20 Grad um die Drehachse beweglich sind. Solche Handgelenkorthesen sind besonders geeignet zur konservativen und postoperativen Therapie von ligamentären Verletzungen einschließlich Rupturen. Die erfindungsgemäße Handgelenkorthese zeichnet sich dadurch aus, dass sie eine Bewegungsfreiheit der Hand relativ zum Unterarm erlaubt, wobei diese Bewegungsfreiheit derart begrenzt ist, dass die Sehnen und Bänder im Wesentlichen nicht belastet werden.

Im Stand der Technik sind eine Vielzahl von Handorthesen bekannt. Entsprechende Handorthesen finden sowohl bei der konservativen Therapie als auch der postoperativen Therapie Anwendung. Handgelenkorthesen dienen zur Stabilisation des Handgelenks insbesondere zur ligamentären Stabilisation des Handgelenks. Das Handgelenk wird, unterstützt von diversen Handmuskeln, ligamentär stabilisiert. Dabei wird von einem muskulären Dreieck gebildet durch die entsprechenden Muskeln gesprochen.

Der Karpus wird funktionell als rein unter Spannung stehender Ring betrachtet. Dabei wird die Spannung von einem komplexen Bandapparat aufgebaut, der die Bewegungsrichtung der Handwurzelknochen führt und den Bewegungsumfang beschränkt. Das Bandsystem wird in extrinsische, extraartikuläre Bänder, die fest mit der Gelenkkapsel verwachsen sind, und intrinsische, interartikuläre Bänder (z.B. das Ligamentum scapholunatum, SL-Band, und Ligamentum lunotriquetrum, LT-Band) zwischen den Handwurzelknochen mit kürzerer Faserlänge eingeteilt.

Verletzungen des Karpus können vielfältige Gründe aufweisen: Frakturen der Handwurzelknochen; ligamentäre Verletzungen des Karpus; chronische Überbelastung; Chondrokalzinose; Hemochromatose; rheumatoide Arthritis; neurogene Erkrankungen.

Eine wesentliche Ursache der karpalen Instabilität sind also ligamentäre Verletzungen des Karpus. Hierzu zählt unter anderem die Verletzung des skapholunären Bandes. Bei einer ligamentären Instabilität, ab Stadium 2, kommt es nicht nur zu einer mechanischen Gefügestörung zwischen den Handwurzelknochen selbst, sondern auch zu einem Ungleichgewicht innerhalb des betreffenden muskulären Dreieckspunkt. Während bei leichten Bandrupturen, wie im Stadium 1, die Muskulatur innerhalb des betreffenden Winkels kompensatorisch die Richtungskräfte erhalten bzw. durch gezielte Stabilisierungsübungen im betreffenden Winkel für den Erhalt dieser Kräfte beitragen können sind bei Instabilitäten ab Stadium 2 weitere Maßnahmen notwendig, um die Stabilität des Handgelenks wieder zu verbessern.

Durch das Aufsprengen der stabilen Ringsystems kommen die natürlichen Bewegungstendenzen von Lunatum und Skaphoid vermehrt zum Tragen. Aufgrund der Anatomie des Handgelenks hat das Lunatum die Tendenz, nach palmar aus dem Karpus zu gleiten und gleichzeitig nach dorsal in Extension zu rotieren. Seine Extension im Vergleich zur Radiuslänge wird als DISI-Position bezeichnet (dorsal intercalated segment instability).

Bei SL-Teilrupturen wird entsprechend die DISI-Position bestimmt, um den Grad bzw. das Stadium der Verletzung zu bestimmen. Eine SL-Bandruptur wird in drei Schweregrade (Grad I, Grad II, Grad III) eingeteilt. Gleiches gilt für Rupturen des LT-Bandes. Üblicherweise wird ab Stadium 2 operativ behandelt.

Zur Unterstützung der postoperativen Maßnahmen oder bei konservativer Therapie zur Teilruhigstellung werden Handorthesen eingesetzt. Üblicherweise findet hierbei eine vollständige Ruhigstellung des Handgelenks statt. Das Problem hierbei ist allerdings, dass durch die vollständige Ruhigstellung der Bewegungsablauf und die Muskulatur negativ beeinflusst werden.

Aus der US 2011/0077569 A1 ist eine Handgelenkorthese bekannt, die lediglich ein Band im Bereich der inneren Handfläche aufweist. Eine Fixierung oder Immobilisierung des Daumens ist nicht beschrieben. Die Drehachse ist nicht am Handgelenk angeordnet.

Aufgabe der vorliegenden Erfindung ist es, eine Handgelenkorthese bereitzustellen, die sowohl konservativ als auch postoperativ in handtherapeutischen Behandlungsmethoden eingesetzt werden kann und dabei den Heilungsprozess z.B. nach Ruptur des SL-Bandes erlaubt bzw. unterstützt. Insbesondere eignet sich diese Handorthese auch zur konservativen Therapie z.B. bei Radiusfraktur oder -ruptur, wenn sie rechtzeitig, z.B. innerhalb von zwei Wochen nach Trauma, begonnen wird.

Erfindungsgemäß wird diese Aufgabe durch eine Handgelenkorthese mit den Merkmalen des Anspruchs 1 gelöst. Vorteilhafte Ausgestaltungen und Weiterbildungen der Erfindung sind in den Unteransprüchen aufgeführt. Die erfindungsgemäße Handgelenkorthese eignet sich insbesondere zur Verwendung bei der Behandlung von ligamentären Verletzungen einschließlich ligamentären Rupturen.

Weiterhin wird ein System für eine Handgelenkorthese bereitgestellt zur Ausbildung einer erfindungsgemäßen Handgelenkorthese.

Die erfindungsgemäße Handgelenkorthese ist eine mit einer formstabilen Unterarmschiene, die um den an ein Handgelenk angrenzenden Unterarm anlegbar ist, und zumindest eine, wie zumindest zwei, Unterarmschienenverschlusseinrichtung aufweist, mit der die Unterarmschiene an dem Unterarm festlegbar ist. Weiterhin weist die erfindungsgemäße Handgelenkorthese eine formstabile palmar anlegbare Handschiene mit einer Öffnung für den Daumen und mindestens einer Handschienenverschlusseinrichtung auf, mit der diese Handschiene an der Hand, insbesondere Mittelhand, festlegbar ist, wobei die Unterarmschiene und die Handschiene teilweise überlappend angeordnet sind. Die Öffnung des Daumens ist bevorzugt derart ausgebildet, dass das Daumensattelgelenk immobilisiert ist. Die Unterarmschiene und die Handschiene sind erfindungsgemäß um eine Drehachse drehgelenkig miteinander verbunden. Die erfindungsgemäße Handgelenkorthese zeichnet sich dadurch aus, dass die Schienen relativ zueinander um einen Drehwinkel von maximal 20 Grad zueinander um die Drehachse beweglich sind.

Die Erfindung sieht dabei vor, dass durch die Schienen einerseits eine Stabilisierung des Handgelenkes erfolgt und das Handgelenk in seiner Bewegung auf den genannten Drehwinkel beschränkt ist. Andererseits erlaubt die erfindungsgemäße Handgelenkorthese aber auch eine Bewegung innerhalb dieses genannten Drehwinkels, um die Muskulatur und den Bandapparat des Handgelenkes zu trainieren und das Handgelenk dabei muskulär zu stabilisieren.

Die erfindungsgemäße Handgelenkorthese nutzt dabei bevorzugt das Modell der muskulär stabilisierenden Stabilisation des Handgelenks bzw. des Stabilisatonsdreiecks gebildet durch die Muskulatur des Handgelenks, hier insbesondere M. extensor carpi radialis longus et brevis, M. abduktor pollicis longus und M. extensor carpi ulnaris sowie M. flexor digitorum superficialis et profundus, M. flexor carpi ulnaris und M. flexor carpi radialis sowie dem M. extensor digitorum communis. Hier spielt auch das TFCC, trianguläre fibrokartilaginäre Complex, Discus triangularis, eine Rolle.

Die erfindungsgemäße Handgelenkorthese zeichnet sich durch den genannten freien Bewegungswinkel bzw. Bewegungsradius in allen Freiheitsgraden des Handgelenkes aus (z.B. postoperativ adaptiert) bei einer DISI (mit und ohne Radiusfraktur). Eine DISI musste bisher auch ohne Fraktur bei einer Schädigung von Grad II operativ versorgt werden, mit der erfindungsgemäßen Orthese ist es nun möglich solche Schädigungen auch ohne Operation zu therapieren.

Mit Hilfe der erfindungsgemäßen Handgelenksorthese ist es möglich, die sogenannte "dart-throwing motion" durchzuführen. Hierbei handelt es sich um eine schräge Handgelenksbewegung von Radialextension bis Ulnarflexion in schräger Drehachse. Der Vorteil dieser schrägen Achse liegt darin, dass für die resultierende schräge Handgelenksbewegung das Mediocarpalgelenk im größeren Umfang genutzt wird als das Radiocarpalgelenk und sich die proximale Handwurzelreihe als relative Einheit in der schrägen (zwischen der Frontal- und Sagittalebene gelegenen) Ebene bewegt. Ist die radioulnare Achse gerade ausgerichtet, wie aus dem Stand der Technik bekannt, erlaubt sie ausschließlich axiale Flexions- und Extensionsbewegungen des Handgelenkes in der Sagittalebene. Diese finden zu einem großen Teil auch im Radiocarpalgelenk statt, wodurch ligamentäre Pathologien, wie beispielsweise eine SL-Bandverletzung damit nicht dynamisch nachbehandelt werden können. Dieses kräftigt die Muskulatur entsprechend. Dabei ist es wichtig, dass die Bänder, wie das SL- oder das LT-Band, nur minimal belastet werden. So zeigten Studien, dass beim Dart werfen die Bewegung in der proximalen Karpalreihe minimiert wird. Therapeutische Protokolle zeigen beim Dart werfen auf, dass die Bewegung begrenzt wird und den Heilungsprozess somit unterstützen kann.

Die erfindungsgemäße Handgelenkorthese lässt eine solche "dart throw motion" zu und unterstützt dabei eine Dorsalextension mit Radialduktion und Palmarflexion mit Ulnarduktion. In einer Ausführungsform ist dabei die Bewegung der Drehachse derart, dass die Ulnarflexion reduziert ist, z.B. dass der maximale Drehwinkel ausgehend von der Nullstellung nur in palmar-radial Richtung möglich ist.

In einer Ausführungsform ist die erfindungsgemäße Handgelenkorthese eine, bei der die Anordnung der Schienen derart ist, dass die Schiene nach Festlegung der Hand in der Handschiene und des Unterarms an der Unterarmschiene relativ zu der Nullstellung der Hand zum Unterarm einen maximalen Drehwinkel um die Drehachse von 10 Grad in beide Drehrichtungen erlaubt. Alternativ ist der maximale Drehwinkel einer von 20 Grad, wie 10 Grad, in palmar-radial Richtung ausgehend von der Nullstellung. In einer Ausführungsform ist der maximale Drehwinkel um die Drehachse von 5 Grad in beide Drehrichtungen möglich. D.h., die Orthese lässt einen Bewegungsradius von ca. 5 Grad in allen Freiheitsgraden des Handgelenks zu; dadurch ist eine muskuläre Stabilisation möglich, ohne das die Bänder, wie das SL-Band oder das LT-Band, aber auch der TFCC beansprucht werden. Es zeigte sich, dass bei geringen Bewegungsradien, d.h. geringen Drehwinkeln, eine Belastung der Bänder noch nicht stattfindet, während die Muskulatur trainiert wird. Bevorzugt ist dabei eine reduzierte Ulnarflexion, die nur noch bis zur Nullstellung möglich ist.

In einer Ausführungsform sind die Unterarmschiene und/oder die Handschiene aus einem thermoplastischen Material ausgebildet. Dieses thermoplastische Material erlaubt die individuelle Anpassung der Schienen an das Individuum und ggf. ein nachjustieren hiervon nach einiger Zeit. Dadurch ist eine individuelle Anpassung der erfindungsgemäßen Handorthese an den Träger oder die Trägerin möglich.

Die Unterarmschiene ist insbesondere eine palmare Unterarmschiene, die sich in der Länge über dreiviertel des Unterarms erstreckt und mindestens die halbe Unterarmhöhe umschließt. Der distale Teil verläuft palmar bis zur Beugehandfalte, dorsal bis proximal der MCPs mit Einschluss des CMC 1-Gelenks, wobei MCP und IP frei liegen.

Die Verschlusseinrichtungen an der Unterarmschiene und/oder Handschiene sind z.B. individuell einstellbare Verschlusseinrichtungen, z.B. in Form von Klettbändern. Z.B. können zwei bis drei Klettbänder vorliegen wobei eins hiervon am proximalen Ende angeordnet ist und das andere proximal zum Handgelenk. Hierdurch ist die Unterarmschiene fest anlegbar an dem Unterarm. Die Handschiene weist mindestens eine Handschienenverschlusseinrichtung auf, die distal über den Handrücken nach medial schließend verläuft.

Die Verschlusseinrichtungen erlauben ein Festlegen der Schienen an die Hand- bzw. dem Unterarm des Individuums.

Die Schienen sind dabei in einer Ausführungsform überlappend ausgebildet, insbesondere überlappend die beiden Schienen radial-palmar und dorsal-ulnar.

Die Drehachse der Schienen liegt dabei in einer Ausführungsform am Radius des Handgelenks. Das Drehgelenk, die Drehachse, kann in schräger Achse, nämlich von palmar-radial nach dorsal-ulnar ausgebildet sein, so dass eine Dorsalextension mit Radialduktion und Palmarflexion mit Ulnarduktion möglich ist. Die Handgelenkorthese ist derart ausgebildet, dass sie eine schräge Achse wie das Handgelenk aufweist. Dadurch ist eine gekoppelte Bewegung von Orthese und Handgelenk möglich.

Wie ausgeführt sind die beiden Schienen auf Höhe der Handgelenksbeugefalte bevorzugt überlappend, angeordnet. Dabei kann zum Schutz vor Hauteinklemmung in Flexion ein, ggf. dünnes, Polster von innen in die Schiene eingebracht sein, die sich von der Handschiene bis zur Unterarmschiene erstreckt. In einer Ausführungsform überlappen die Schienenteile radial-palmar und dorsal-ulnar, um das entsprechende Drehgelenk mit der entsprechenden Drehachse auszubilden, wobei das Drehgelenk z.B. in schräger Achse angeordnet ist.

In einer Ausführungsform wird der Drehwinkel durch Anschlagspositionen an den Schienen begrenzt. Eine Form der Anschlagsposition ist palmar die Begrenzung des Drehwinkels durch ein teilweises Anschlagen der Stirnseiten der Schienen. Die Handgelenkorthese kann weiterhin eine Begrenzung des Drehwinkels dorsal durch Hinterschneiden einer Schiene relativ zu der zweiten Schiene aufweisen. Die Anschlagspositionen der Schienen zueinander sind derart ausgebildet, dass ein Drehwinkel um die Drehachse in angegebenen Maße möglich ist. Dem Fachmann sind geeignete Möglichkeiten zur Ausbildung entsprechender Anschläge bekannt. So kann eine Begrenzung bzw. die Ausbildung der Anschlagsposition auch durch entsprechende Vorsprünge im oder am Drehgelenk erfolgen. In einer Ausführungsform kann die Gelenkachse dabei durch Verpressung der beiden Schienen ausgebildet werden.

Die Gelenke können durch geeignete Mittel ausgebildet werden. So können zur Ausbildung des Gelenks entsprechende Verschraubungen etc. durchgeführt werden. Zum Schutz der Haut werden solche Verschraubungen gegebenenfalls entsprechend abgepolstert, um eine Beschädigung der Haut zu vermeiden.

In einer Ausführungsform ist die Drehachse, das Drehgelenk in ihrer Rotationsbewegung gehindert. Das heißt, die das Drehgelenk bzw. Drehachse ausbildende Unterarmschiene und die Handschiene sind zur Verhinderung einer Rotationsbewegung um die Drehachse feststellbar, wie arretierbar. Dem Fachmann sind geeignete Mittel zum feststellen, wie zu arretieren, bekannt.

In einem weiteren Aspekt richtet sich die vorliegende Erfindung auf ein System für eine Handgelenkorthese umfassend eine erste formstabile Unterarmschiene, die um den an das Gelenk angrenzende Unterarm anlegbar ist und mindestens eine, wie mindestens zwei, Verschlusseinrichtung aufweist, mit der die Unterarmschiene an dem Unterarm festlegbar ist und eine zweite formstabile Handschiene, die palmar anlegbar ist mit einer Öffnung für den Daumen, bevorzugt derart, dass das Daumensattelgelenk immobilisiert ist, und mindestens einer Verschlusseinrichtung, mit der diese Handschiene an der Hand festlegbar ist, diese Schienen sind miteinander verbindbar zur Ausbildung einer erfindungsgemäßen Handgelenkorthese.

Das erfindungsgemäße System kann dabei entsprechende Anleitungen zum Zusammenbau und zum Anpassen der Handgelenkorthese enthalten.

Schließlich wird die Verwendung der erfindungsgemäßen Handorthese zur Behandlung von ligamentären Verletzungen einschließlich ligamentären Rupturen bereitgestellt. Hierbei handelt es sich insbesondere um solche mit Schädigung des SL-Bandes und des LT-Bandes aber auch des TFCC. Die Handgelenkorthese ist insbesondere geeignet bei einer Ruptur ohne Fraktur mit einer Grad II Schädigung. Die Handgelenkorthese eignet sich zur Verwendung bei konservativer Therapie als auch bei postoperativer Therapie.

Im Folgenden wird die Erfindung unter Bezugnahme auf die beigefügten Figuren näher erläutert.

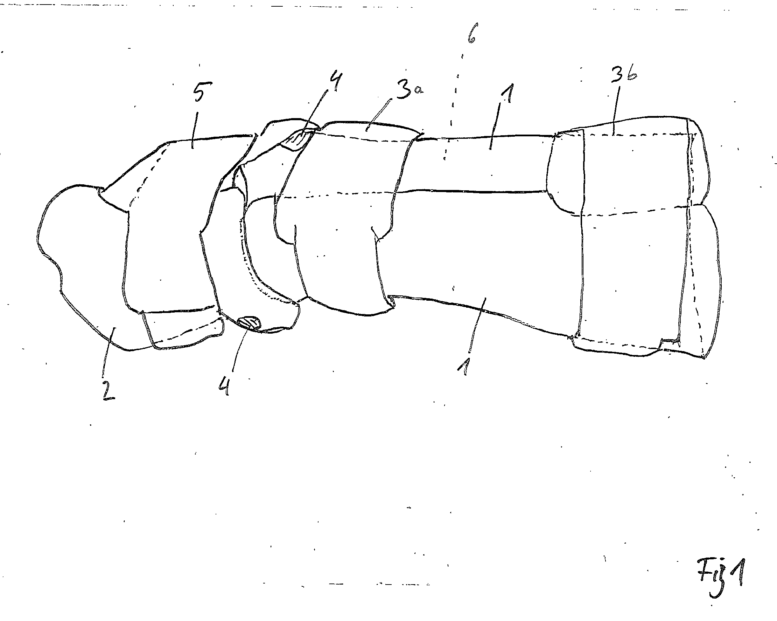

Dabei zeigt die Figur 1 eine Ansicht einer erfindungsgemäßen Handgelenkorthese.

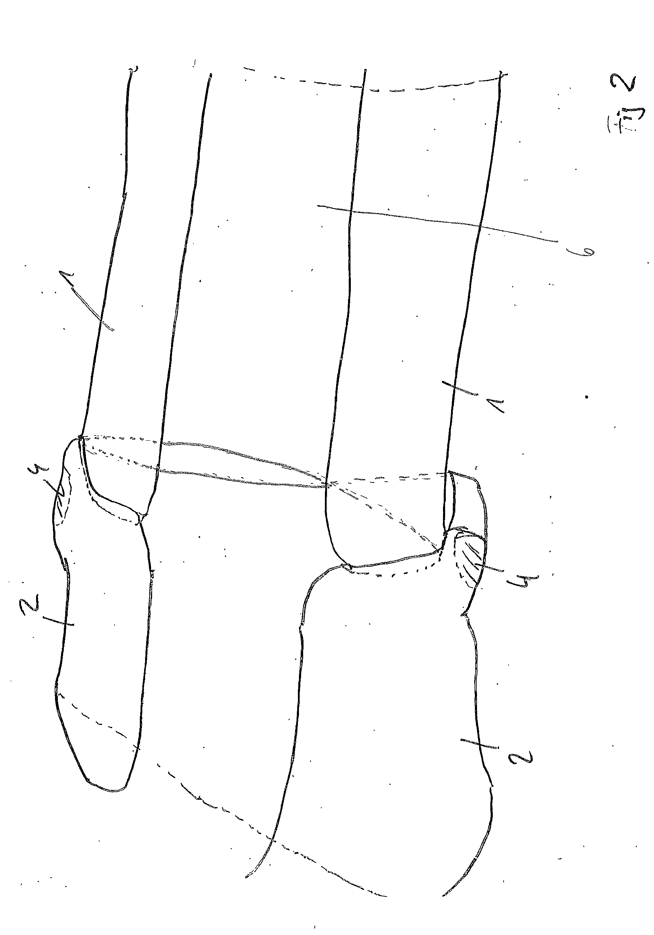

Die Figur 2 zeigt eine weitere Ansicht der erfindungsgemäßen Handgelenksorthese, wobei die Verschlusseinrichtungen nicht dargestellt sind.

In der Figur 1 ist perspektivisch eine erfindungsgemäße Handgelenkorthese dargestellt. Die Unterarmschiene 1 ist dabei eine als palmare Unterarmschiene ausgebildet, die sich über 3/4 des Umfangs erstreckt. Der mit 6 bezeichnete Bereich ist dabei ausgespart und erlaubt eine entsprechend individuelle Anpassung an den Unterarm. Die Unterarmschiene weist zwei Unterarmschienenverschlusseinrichtungen 3 auf. Eine ist dabei am proximalen Ende angeordnet (3b) während die zweite proximal zum Handgelenk angeordnet ist (3a). Die Handschiene 2 ist mit einer Handschienenverschlusseinrichtung 5 ausgestattet. Nicht dargestellt ist die Öffnung für den Daumen in der Handschiene. Die Handschiene 2 ist mit der Unterarmschiene 1 überlappend angeordnet. Dabei weisen die Schienen 1 und 2 eine Drehachse 4 auf. Diese Drehachse 4 ist wie dargestellt palmar-radial nach dorsal-ulnar ausgebildet. Hierdurch sind Dorsalextension mit Radialduktion und Palmarflexion mit Ulnarduktion möglich.

Über das Drehgelenk sind Unterarmschiene und Handschiene bewegbar. Dabei ist ein Drehwinkel zwischen diesen beiden Schienen relativ zueinander um maximal 20 Grad eingestellt, bevorzugt maximal 10 Grad. Durch die Überlappung der Handschiene 2 mit der Unterarmschiene 1 ist der Drehwinkel begrenzt.

Figur 2 ist eine weitere Darstellung der erfindungsgemäßen Handgelenkorthese. Dargestellt ist die Unterarmschiene 1 und die Handschiene 2 mit dem Drehwinkel 4, der auch vorliegend wieder palmar-radial nach dorsal-ulnar ausgebildet ist. 6 bezeichnet den freien dorsalen Bereich der Unterarmschiene und der Handgelenksschiene. Die Unterarmschienenverschlusseinrichtung 3 ist ebenso wie die Handschienenverschlusseinrichtung 5 nicht dargestellt. Deutlich ist die Überlappung der Unterarmschiene 1 mit der Handschiene 2 zu erkennen. Vorliegend ist die Ausbildung des Drehgelenks 4 durch Verpressung der Unterarmschiene mit der Handgelenkschiene 2 möglich.

## Patentansprüche

1. Handgelenkorthese mit einer formstabilen Unterarmschiene (1), die um den an ein Handgelenk angrenzenden Unterarm anlegbar ist und mindestens eine, wie mindestens zwei, Unterarmschienenverschlusseinrichtungen (3) aufweist, mit der die Unterarmschiene (1) an dem Unterarm festlegbar ist, und mit einer formstabilen palmar anlegbaren Handschiene (2) mit einer Öffnung für den Daumen, bevorzugt derart, dass das Daumensattelgelenk immobilisiert ist, und mindestens einer Handschienenverschlusseinrichtung (5), mit der diese Handschiene (2) an der Hand festlegbar ist, wobei die Unterarmschiene (1) und die Handschiene (2) teilweise überlappend angeordnet sind, und wobei die Unterarmschiene (1) und die Handschiene (2) um eine Drehachse (4) drehgelenkig miteinander verbunden sind, **dadurch gekennzeichnet, dass** die Schienen (1, 2) relativ zueinander um einen Drehwinkel von maximal 20 Grad zueinander um die Drehachse (4) beweglich sind und die Drehachse (4) am Radius des Handgelenks liegt.

2. Handgelenkorthese nach Anspruch 1, **dadurch gekennzeichnet, dass** die Anordnung der Schienen (1, 2) derart ist, dass die Schienen nach Festlegung der Hand in der Handschiene (2) und des Unterarmes in der Unterarmschiene (1) relativ zu der Nullstellung der Hand zum Unterarm i) einen maximalen Drehwinkel um die Drehachse von 10 Grad in beide Drehrichtungen erlauben oder ii) einen maximalen Drehwinkel um die Drehachse von 20 Grad in palmar-radial Richtung.

3. Handgelenkorthese nach Anspruch 1 oder 2, wobei der maximale Drehwinkel um die Drehachse (4) von i) 5 Grad in beide Drehrichtungen oder ii) von 10 Grad in palmar-radial Richtung beträgt.

4. Handgelenkorthese nach einem der Ansprüche 1 oder 2, **dadurch gekennzeichnet, dass** die Unterarmschiene (1) und/oder die Handschiene aus einem thermoplastischen Material ausgebildet ist.

5. Handgelenkorthese nach einem der vorherigen Ansprüche, **dadurch gekennzeichnet, dass** die beiden Schienen (1, 2) radial-palmar und dorsalulnar überlappen.

6. Handgelenkorthese nach einem der vorherigen Ansprüche, **dadurch gekennzeichnet dass** diese in ihrer Drehachse feststellbar ist.

7. Handgelenkorthese nach einem der vorherigen Ansprüche, **dadurch gekennzeichnet, dass** der Drehwinkel durch Anschlagspositionen an den Schienen (1, 2) begrenzt ist.

8. Handgelenkorthese nach einem der vorherigen Ansprüche, **dadurch gekennzeichnet, dass** palmar die Begrenzung des Drehwinkels durch ein Anschlagen der Stirnseiten der Schienen (1, 2) erfolgt.

9. Handgelenkorthese nach einem der vorherigen Ansprüche, **dadurch gekennzeichnet, dass** die Begrenzung des Drehwinkels dorsal durch Hinterschneidung einer Schiene relativ zu der zweiten Schiene erfolgt.

10. Handgelenkorthese nach einem der vorherigen Ansprüche, **dadurch gekennzeichnet, dass** die Drehachse (4) von palmar-radial nach dorsal-ulnar angeordnet ist.

11. Handgelenkorthese nach einem der vorherigen Ansprüche, **dadurch gekennzeichnet, dass** die Drehachse (4) ausgebildet ist durch Pressspannung der beiden Schienen (1, 2).

12. Handgelenkorthese nach einem der vorherigen Ansprüche, **dadurch gekennzeichnet dass** der Daumen im Daumensattelgelenk durch die Schiene (2) immobilisiert ist, die Drehachse gegebenenfalls feststellbar ist, und die Ulnarflexion nur bis zur Nullstellung des Handgelenks möglich ist.

13. System für eine Handgelenkorthese umfassend eine erste formstabile Unterarmschiene (1), die um den an das Gelenk angrenzenden Unterarm anlegbar ist und mindestens eine, wie mindestens zwei, Verschlusseinrichtung (3) aufweist, mit der die Unterarmschiene (1) an dem Unterarm festlegbar ist und eine zweite formstabile Handschiene (3), die palmar anlegbar ist mit einer Öffnung für den Daumen, bevorzugt derart, dass das Daumensattelgelenk immobilisiert ist, und mindestens einer Verschlusseinrichtung (5), mit der diese Handschiene (1) an der Hand festlegbar ist, die miteinander verbindbar sind zur Ausbildung einer Handprothese nach einem der Ansprüche 1 bis 12.

14. Verwendung einer Handgelenkorthese nach einem der Ansprüche 1 bis 12 zur Behandlung ligamentären Verletzungen einschließlich Rupturen.
